# FASCICULE DE BREVET EUROPEEN

(11) **EP 0 500 409 B1**
(45) Date de publication et mention de la délivrance du brevet: **20.03.1996**
(21) Numéro de dépôt: 92400269.4
(22) Date de dépôt: 03.02.1992
(51) Int. Cl.: C07D 239/36, C07D 403/10, C07D 409/04, C07D 401/14, C07D 417/14, C07D 405/14, A61K 31/505

(54) **Dérivés de 4-pyrimidinones, leur préparation et leur application en thérapeutique**
4-Pyrimidinonderivate, ihre Herstellung und ihre Anwendung in der Therapie
4-Pyrimidinone derivatives, their preparation and their application in therapy

(30) Priorité: 20.02.1991 FR 9102032
(43) Date de publication de la demande: 26.08.1992
(73) Titulaire: SYNTHELABO, F-92350 Le Plessis Robinson (FR)
(72) Inventeur: Hoornaert, Christian, F-75013 Paris (FR); Daumas, Marc, F-75013 Paris (FR); Aletru, Michel, F-75020 Paris (FR); Muller, Jean-Claude, F-91390 Morsang sur Orge (FR)
(74) Mandataire: Thouret-Lemaitre, Elisabeth

(56) Documents cités:
- EP-A- 424 317
- EP-A- 465 323
- EP-A- 0 401 030
- EP-A- 0 407 342
- WO-A-91/15209
- JOURNAL OF MEDICINAL AND PHARMACEUTICAL CHEMISTRY, vol. 5, no. 6, 27 Novembre 1962, EASTON, US, pages 1103 - 1123; B. ROTH ET AL.: '5-Benzyl-2.4- diaminopyrimidines as antibacterial agents. I. Synthesis'

## Description

La présente invention a pour objet des dérivés de 4-pyrimidinones, leur préparation et leur application en thérapeutique.

Plusieurs demandes de brevets publiées décrivent des dérivés de pyrimidinones actifs comme antagonistes d'angiotensine II et utiles dans diverses formes de pathologie hypertensive.

Ainsi la demande de brevet européen EP 0424317 déposée le 10 octobre 1990 (priorité du 19 octobre 1989) et publiée le 24 avril 1991 décrit des dérivés de pyrimidine de formule (a) 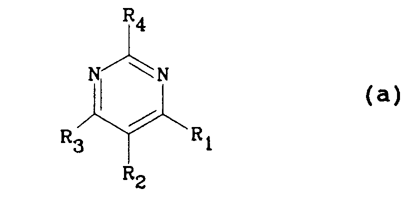
dans laquelle R₁ peut représenter un groupe hydroxy, R₂ un groupe biphényl-4-ylméthyle substitué par un groupe -CO₂H ou tétrazol-5-yle, R₃ un groupe butyle et R₄ un groupe (C₁-C₄)alkyle.

La demande de brevet européen EP 0465323 déposée le 28 juin 1991 (priorité du 29 novembre 1990) et publiée le 8 janvier 1992 décrit des dérivés de formule (b) 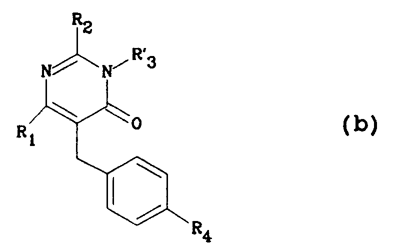
dans laquelle R₁ peut représenter un groupe butyle, R₂ un groupe (C₁-C₄)alkyle ou un noyau aromatique, R'₃ un atome d'hydrogène et R₄ un groupe phényle substitué par un groupe -CO₂H ou tétrazol-5-yle.

La demande de brevet WO 91/15209 déposée le 27 mars 1991 (priorité 30 mars 1990) publiée le 17 octobre 1991 décrit entre autres des dérivés de formule (c) 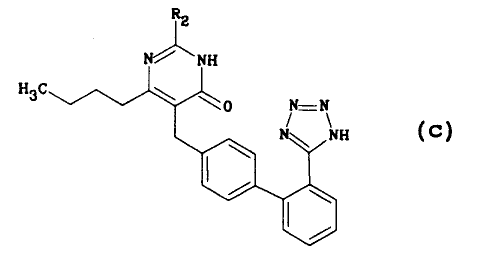
dans laquelle R₂ peut-être un groupe (C₁-C₄)alkyle éventuellement substitué par un groupe aryle.

La demande de brevet européen EP 0407342 décrit des dérivés de pyrimidine de formule (d) 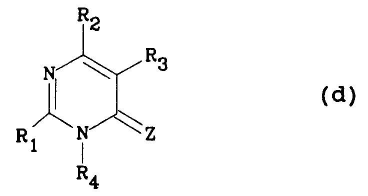
dans laquelle R₄ peut représenter un groupe biphényl-4-ylméthyle substitué par un groupe -CO₂H ou tétrazol-5-yle et Z un atome d'oxygène.

La présente invention décrit de nouveaux dérivés de 4-pyrimidinones actifs comme antagonistes d'angiotensine II et utiles dans diverses formes de pathologie antihypertensive.

Les composés de l'invention répondent à la formule générale (I) 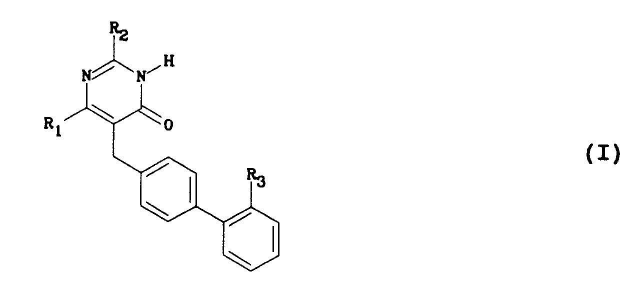
dans laquelle
R₁ représente un groupe droit butyle,
R₂ représente un groupe phénylméthyle ou phényléthyle et
R₃ représente un groupe CO₂H ou 1*H*-tétrazol-5-yle.

Les composés de l'invention peuvent se présenter sous les trois formes tautomères 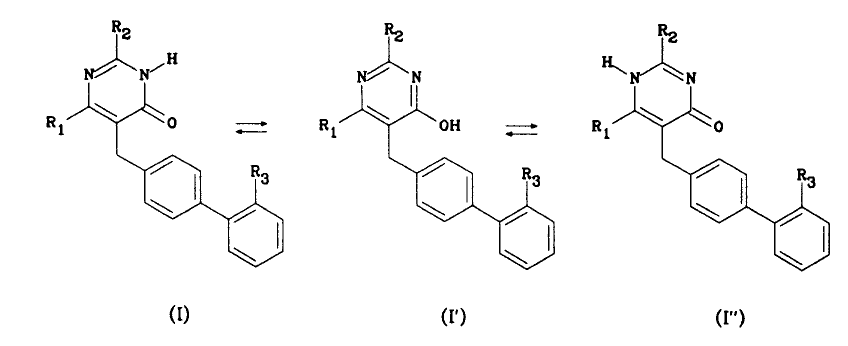
qui font partie de l'invention.
Les composés peuvent se présenter sous forme libre ou sous la forme de sels organiques ou inorganiques pharmaceutiquement acceptables.

Les composés de l'invention peuvent être préparés suivant le schéma suivant: 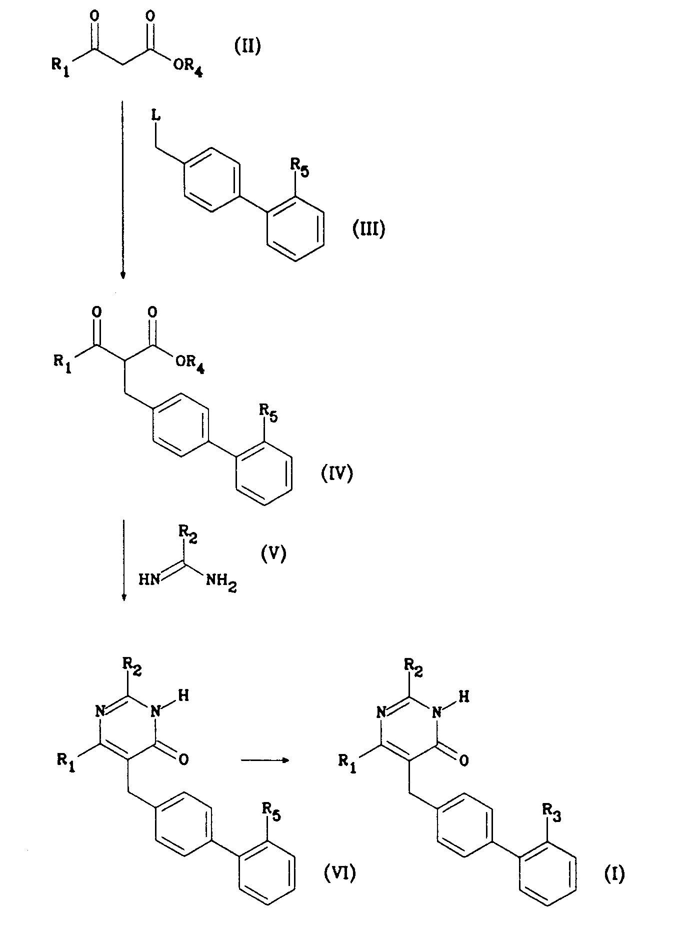
Dans une première étape, on fait réagir un β-cétoester de formule générale (II) dans laquelle R₁ est tel que défini ci-dessus et R₄ représente un groupe méthyle ou éthyle, avec un dérivé de formule générale (III) dans laquelle L représente un groupe partant tel que chloro, bromo, iodo, p-toluènesulfonyloxy ou méthanesulfonyloxy et R₅ représente soit un groupe ester carboxylique CO₂R₆, où R₆ est un groupe méthyle, éthyle, 1,1-diméthyléthyle ou benzyle, soit un groupe 1*H*-tétrazol-5-yle protégé, par exemple, par un substituant triphénylméthyle ou 1,1-diméthyléthyle, pour donner des dérivés de β-cétoester de formule générale (IV) dans laquelle R₁, R₄ et R₅ sont tels que définis ci-dessus. Les dérivés de formule générale (III) sont décrits dans différentes demandes de brevets européens (253310, 291969, 323841, 400835, 400974, 401030). La réaction est conduite dans un solvant tel que le méthanol, l'éthanol, le 1,1-diméthyléthanol ou le diméthylformamide à une température comprise entre - 20°C et + 80°C, en présence d'une base telle que le méthylate de sodium, le 1,1-diméthyléthylate de potassium ou l'hydrure de sodium et éventuellement en présence d'un catalyseur tel que le bromure ou l'iodure de lithium, de magnésium ou de zinc.

Dans une deuxième étape, on fait réagir un β-cétoester de formule générale (IV) avec une amidine de formule générale (V) pour obtenir une pyrimidinone de formule générale (VI) dans laquelle R₁, R₂ et R₅ sont tels que définis précédemment. La réaction est conduite en chauffant entre 40°C et 120°C un mélange des deux composés, éventuellement dans un solvant tel que le méthanol, l'éthanol, le butanol ou le toluène et éventuellement en présence d'une base telle que le méthylate de sodium, l'acétate de sodium, le carbonate de potassium, la pyridine, la triéthylamine ou la 4-diméthyl-aminopyridine.

Dans une troisième étape, on réalise la déprotection et/ou la transformation du groupe R₅ en groupe R₃ suivant la nature de ce groupe:
- lorsque R₅ est un ester d'acide carboxylique, on soumet les composés de formule générale (VI) à une hydrolyse acide ou basique pour obtenir les composés de formule générale (I) dans laquelle R₁ et R₂ sont tels que définis précédemment et R₃ est un groupe CO₂H,
- lorsque R₅ est le groupe triphénylméthyl-1*H*-tétrazol-5-yle ou le groupe 1,1-diméthyléthyl-1*H*-tétrazol-5-yle, on soumet les composés de formule générale (VI) à une réaction de déprotection en milieu acide, suivant des conditions classiques, pour obtenir les composés de formule générale (I) dans laquelle R₃ est le groupe 1*H*-tétrazol-5-yle et R₁ et R₂ sont tels que définis précédemment.

Les exemples suivants illustrent l'invention. Les numéros renvoient au tableau donné plus loin. Les analyses confirment la structure des composés.

### Exemple 1 (composé no 1) acide 4'-[(6-butyl-2-phénylméthyl-4-oxo-1,4-dihydro-pyrimidin-5-yl)méthyl][1,1'-biphényle]-2-carboxylique.

1.1. 4'-[(2-méthoxycarbonyl)-3-oxoheptyl][1,1'-biphényle]-2-carboxylate de méthyle.
   A une solution de 16 g de 3-oxo-heptanoate de méthyle dans 145 ml de méthanol, refroidie par un bain de glace, on ajoute, sous agitation, une solution de 5,57 g de méthylate de sodium (préparée à partir de 2,36 g de sodium dans 60 ml de méthanol). L'addition terminée, on poursuit l'agitation à la température ambiante pendant une heure puis on refroidit à nouveau par un bain de glace. On ajoute goutte à goutte une solution de 40,12 g de 4'-(bromométhyl)[1,1'-biphényle]-2-carboxylate de méthyle dans 60 ml de méthanol. On laisse le mélange réactionnel sous agitation, à la température ambiante, pendant 24 heures. On le concentre sous pression réduite. On reprend le résidu par du dichlorométhane. On le lave avec une solution aqueuse 1 N d'acide chlorhydrique puis avec de l'eau; on le sèche sur du sulfate de sodium et on l'évapore sous vide pour obtenir 42,4 g de produit, utilisé tel quel dans l'étape suivante.
1.2. acide 4'-[(6-butyl-2-phénylméthyl-4-oxo-1,4-dihydro-pyrimidin-5-yl)méthyl][1,1'-biphényle]-2-carboxylique.
   On chauffe à 95°C, sous argon, pendant 4 heures, un mélange de 0,56 g de benzèneéthanimidamide et de 1,7 g de 4'-[(2-méthoxycarbonyl)-3-oxoheptyl][1,1'biphényle]-2-carboxylate de méthyle. On purifie le produit obtenu par chromatographie sur alumine en éluant par un mélange de dichlorométhane et de méthanol pour obtenir 1,0 g de produit. On redissout ce produit dans un mélange de 30 ml de méthanol et de 3 ml d'eau en présence de 1,0 g d'hydroxyde de sodium. On chauffe au reflux pendant 3 heures puis on concentre sous pression réduite. On reprend le résidu par de l'eau. On lave la phase aqueuse par de l'éther, on la filtre et on l'acidifie par une solution aqueuse d'acide chlorhydrique 3 N. On filtre le précipité et on le recristallise dans le méthanol pour obtenir 0,60 g de composé sous forme de poudre blanche.
   Point de fusion : 219°C

### Exemple 2 (composé no 2) acide 4'-[[6-butyl-2-(2-phényléthyl)-4-oxo-1,4-dihydro-pyrimidin-5-yl]méthyl][1,1'-biphényle]-2-carboxylique.

Suivant l'exemple 1, on chauffe à 95°C, pendant 6 heures, un mélange de 1,0 g de benzènepropanimidamide et de 2,4 g de 4'-(2-méthoxycarbonyl-3-oxoheptyl)[1,1'-biphényle]-2-carboxylate de méthyle.
On purifie le produit obtenu par chromatographie sur colonne de gel de silice en éluant par un mélange de dichlorométhane et de méthanol pour obtenir 1,0 g de produit sous forme de sirop. On redissout ce produit dans un mélange de 30 ml de méthanol et de 3 ml d'eau contenant 1,1 g d'hydroxyde de sodium. On chauffe au reflux pendant 3 heures puis on concentre sous pression réduite. On reprend le résidu par de l'eau. On lave la phase aqueuse par de l'éther puis on l'acidifie par une solution aqueuse d'acide chlorhydrique 3 N. On filtre le précipité et on le recristallise dans le méthanol pour obtenir 0,6 g de composé sous forme de poudre blanche.
Point de fusion : 194°C

### Exemple 3 (composé no 3) 6-butyl-2-(2-phényléthyl)-5-[[2'-(1H-tétrazol-5-yl)[1,1'-biphényl]-4-yl]méthyl]-pyrimidin-4(1H)-one.

3.1. 3-oxo-2-[[2'-(1-triphénylméthyl-1*H*-tétrazol-5-yl)[1,1'-biphényl]-4-yl]méthyl]heptanoate de méthyle.
   A une solution de 0,97 g de 1,1-diméthyléthylate de potassium dans 15 ml de diméthylformamide, refroidie par un bain de glace et sous argon, on ajoute une solution de 1,3 g de 3-oxo-heptanoate de méthyle dans 13 ml de diméthylformamide puis 1,67 g de bromure de lithium et ensuite une solution de 5 g de 5-[4'-(bromométhyl)[1,1'-biphényl]-2-yl]-1-triphénylméthyl-1*H*-tétrazole dans 25 ml de diméthylformamide. On agite le mélange réactionnel pendant 5 heures, à la température ambiante, puis on le dilue dans 300 ml d'éther. On le lave avec deux fois 100 ml d'eau puis avec 100 ml d'une solution aqueuse saturée de chlorure d'ammonium, on le sèche sur du sulfate de magnésium et on l'évapore sous vide pour obtenir 4,60 g de produit sous forme d'huile jaune, utilisée telle quelle dans l'étape suivante.
3.2. 6-butyl-2-(2-phényléthyl)-5-[[2'-(1-triphénylméthyl-1*H*-tétrazol-5-yl)[1,1'-biphényl]-4-yl]méthyl]-pyrimidin-4(1*H*)-one.
   On chauffe à 90°C, sous argon, pendant 2 heures, un mélange de 0,7 g de benzènepropanimidamide et de 3 g du composé précédent. On reprend par 120 ml de dichlorométhane et on lave avec deux fois 50 ml d'une solution aqueuse 1 M de carbonate de potassium puis avec 50 ml d'une solution aqueuse 0,1 N d'acide chlorhydrique. On sèche sur du sulfate de magnésium et on évapore. On purifie le résidu par chromatographie sur colonne de gel de silice en éluant par un mélange de dichlorométhane et de méthanol pour obtenir 0,85 g de composé sous forme de mousse blanchâtre.
3.3. 6-butyl-2-(2-phényléthyl)-5-[[2'-(1*H*-tétrazol-5-yl)[1,1'-biphényl]-4-yl]méthyl]-pyrimidin-4(1*H*)-one.
   On dissout 0,6 g du composé précédent dans un mélange de 5 ml de méthanol et de 3 ml de diéthyléther. On ajoute 2,7 ml d'une solution 1,57 M d'acide chlorhydrique dans le diéthyléther. On laisse 2 heures à la température ambiante puis on évapore à sec. On reprend le résidu par 70 ml d'une solution aqueuse 0,5 N d'hydroxyde de sodium. On lave avec trois fois 30 ml d'éther puis on acidifie par une solution d'acide chlorhydrique. On filtre le précipité formé et on le recristallise dans le méthanol pour obtenir 0,22 g de composé sous forme de poudre blanche.
   Point de fusion : 226-228°C

Le tableau suivant illustre les structures et les propriétés physiques des composés selon l'invention.

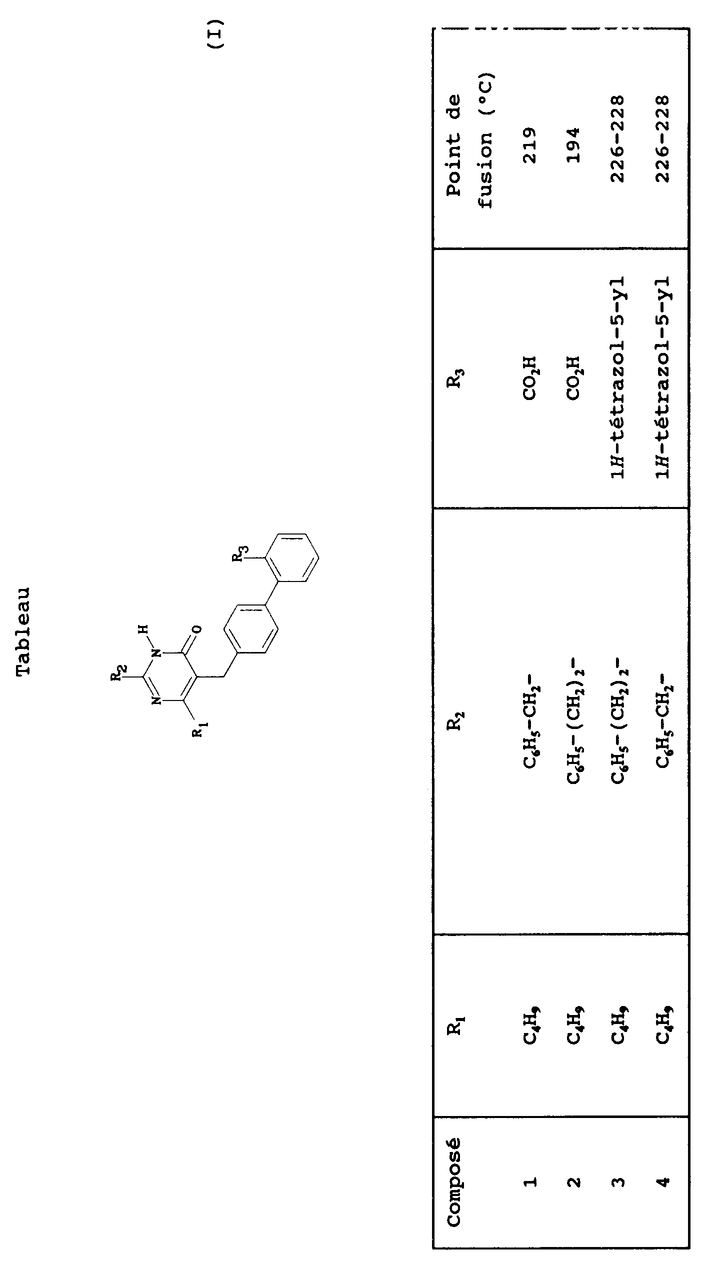

Les composés de l'invention ont fait l'objet d'études pharmacologiques qui ont mis en évidence leurs propriétés antagonistes de l'angiotensine II.

### Essai de liaison (binding) de [³H]-angiotensine II sur la corticosurrénale de lapin.

On utilise des lapins mâles Fauves de Bourgogne de 2 à 3 kg. Après sacrifice des animaux par dislocation cervicale, on excise les glandes surrénales et on dissèque rapidement le cortex à 4°C. On homogénéise le tissu dans 10 ml d'une solution tampon glacée de triS(hydroxyméthyl)aminométhane à 10 mM, contenant du saccharose 0,33 M et de l'acide éthylènediaminetétraacétique 1 mM et dont le pH a été ajusté à 7,4 par de l'acide chlorhydrique, à l'aide d'un appareil Potter électrique à la vitesse de 1200 tours par minute. On ajuste le volume de la préparation à 25 ml avec du tampon tris-saccharose avant de centrifuger 15 min à 1075 x g. On conserve le surnageant, on réhomogénéise le culot dans 10 ml de tampon tris-saccharose, puis on centrifuge comme décrit précédemment On rassemble les deux surnageants et on centrifuge 30 min à 47,800 x g. On reprend le culot par 150 volumes (soit 100 mg de tissu dans 15 ml de tampon) d'une solution de tampon d'incubation contenant: Tris-HCl 50 mM, NaCl 150 mM, acide éthylènediaminetétraacétique 5 mM, 1,25 µg par ml de bacitracine, fluorure de phénylméthylsulfonyl 100 µM et 0,2 % de sérum albumine bovine (pH = 7,4 à 25°C).

On fait ensuite incuber des fractions aliquotes de 100 µl de la suspension contenant les microsomes de corticosurrénales, en présence de [³H]-angiotensine II 2 nM (New England Nuclear, activité spécifique de 61 Ci/mmole), dans un volume final de 0,5 ml de tampon d'incubation, pendant 30 minutes à 25°C. Après incubation, on récupère les microsomes par filtration sur filtres de nitrate de cellulose Millipore HAWP™ 0,45 µm, prétraités par de la sérum albumine bovine à 1 %, et on les lave trois fois avec 5 ml de tampon Tris-HCl glacé. On mesure la quantité de radioactivité liée au tissu et retenue sur les filtres par spectrométrie de scintillation.

La liaison spécifique de la [³H]-angiotensine II est définie comme la quantité de radioactivité fixée sur le filtre qui peut être inhibée par incubation en présence d'angiotensine II non radioactive 1 µM. Cette liaison spécifique représente 90 à 95 % de la quantité totale de radioactivité fixée sur le filtre.
On mesure la liaison spécifique de la [³H]-angiotensine II en présence de différentes concentrations de composés à étudier et on détermine graphiquement la CI₅₀, concentration du composé étudié qui inhibe 50 % de la liaison spécifique de la [³H]-angiotensine II.
Les CI₅₀ des composés selon l'invention se situent entre 5 nM et 10 µM.

### Inhibition de la réponse à l'angiotensine II sur la pression artérielle de rat.

On utilise des rats mâles (Sprague-Dawley, Charles River France) pesant 250 à 280 g, que l'on anesthésie au pentobarbital sodique (55 mg/kg i.p.) et que l'on maintient sous respiration artificielle (Respirateur Harvard™-fréquence de respiration de 70 ml par minute, volume d'air 1 ml par 100 g de poids corporel). On amyèle les animaux à l'aide d'une tige métallique, introduite par l'orbite de l'oeil droit et descendue le long de la colonne vertébrale. On sectionne les nerfs vagues droit et gauche (bivagotomie), on ligature l'artère carotide droite, l'artère carotide gauche étant cathétérisée afin de mesurer la pression artérielle à l'aide d'une cellule de pression (type Statham™ P23Db). On cathétérise une veine fémorale en vue de l'administration de divers composés.
On mesure les variations de pression artérielle moyenne induite par l'angiotensine II administrée par voie intraveineuse à la dose de 0,5 µg/kg. Les composés de l'invention ou le véhicule sont administrés 5 mn (pour les études par voies i.v.) ou 60 minutes (pour les études par voie orale) avant l'administration de l'angiotensine II. Les composés de l'invention sont administrés à des doses allant de 0,01 à 100 mg/kg.
Le pourcentage d'inhibition de la réponse contrôle à l'angiotensine II est utilisé pour apprécier le potentiel antagoniste à l'angiotensine II des composés de l'invention.

Les composés de l'invention peuvent être utilisés pour le traitement de diverses formes de pathologies hypertensives et d'insuffisances coronaire, cardiaque, rénale ou pulmonaire ainsi que pour le traitement du glaucome.
Les composés de l'invention peuvent également être utilisés en association avec d'autres substances à activité cardiovasculaire, telles que les diurétiques, les α-bloquants, les β-bloquants, les antagonistes calciques ou les inhibiteurs de l'enzyme de conversion de l'angiotensine I.

Les composés de l'invention peuvent être présentés sous toutes formes pharmaceutiques appropriées au traitement pour l'administration orale, parentérale, intramusculaire ou rectale : comprimés, capsules, gélules, solutions ou suspensions stériles, suppositoires etc...

Pour le traitement du glaucome, les composés de l'invention peuvent être présentés sous la forme de comprimés, gélules, solutions injectables ou formulations oculaires topiques.

Les composés de l'invention peuvent être administrés aux patients en une quantité pouvant aller de 1 à 1000 mg par jour et par patient, en une ou plusieurs doses.

## Revendications (Revendications pour l'(les) Etat(s) contractant(s) suivant(s): AT, BE, CH, DK, DE, FR, GB, IT, LI, LU, MC, NL, PT, SE)

1. Composés, existant sous trois formes tautomères, répondant aux formules générales (I), (I') et (I'') 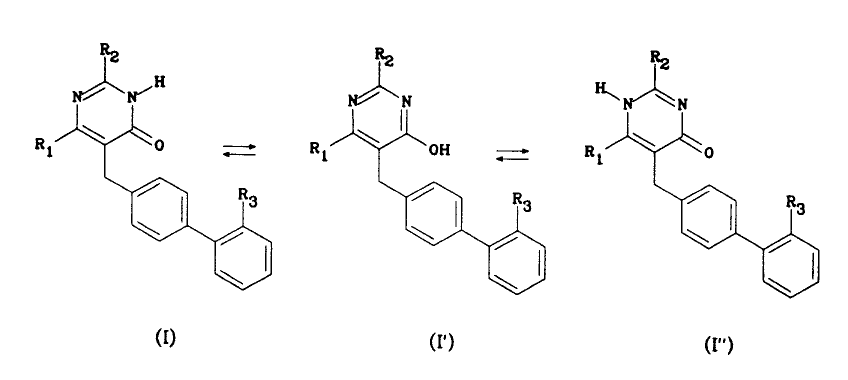 dans lesquelles
R₁ représente un groupe droit butyle,
R₂ représente un groupe phénylméthyle ou phényléthyle, et
R₃ représente un groupe CO₂H ou 1*H*-tétrazol-5-yle,
ainsi que leurs sels organiques ou inorganiques pharmaceutiquement acceptables.

2. Le 6-butyl-2-(2-phényléthyl)-5-[[2'-(1*H*-tétrazol-5-yl)[1,1'-biphényl]-4-yl]méthyl]-pyrimidin-4(1*H*)-one ainsi que ses sels organiques ou inorganiques pharmaceutiquement acceptables, selon la revendication 1.

3. Procédé de préparation des composés selon la revendication 1, procédé caractérisé en ce que l'on fait réagir un β-cétoester de formule générale (II) 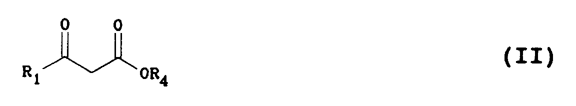 dans laquelle R₁ est tel que défini dans la revendication 1, et R₄ représente un groupe méthyle ou éthyle, avec un dérivé de formule générale (III) 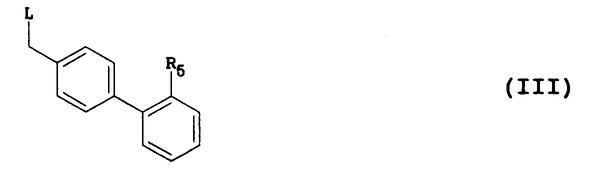 dans laquelle
L représente un groupe partant et R₅ représente soit un groupe CO₂R₆, où R₆ est un groupe méthyle, éthyle, 1,1-diméthyléthyle ou benzyle,
soit un groupe 1*H*-tétrazol-5-yle protégé,
pour obtenir un composé de formule générale (IV) 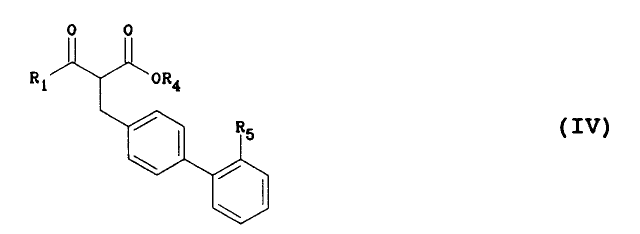 dans laquelle R₁, R₄ et R₅ sont tels que définis dans la revendication 1 et ci-dessus,
composé que l'on fait réagir avec une amidine de formule générale (V) 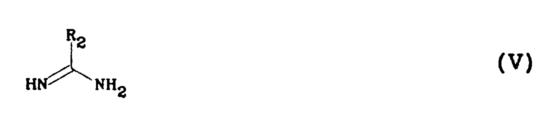 dans laquelle R₂ est tel que défini dans la revendication 1, pour obtenir un composé de formule générale (VI) 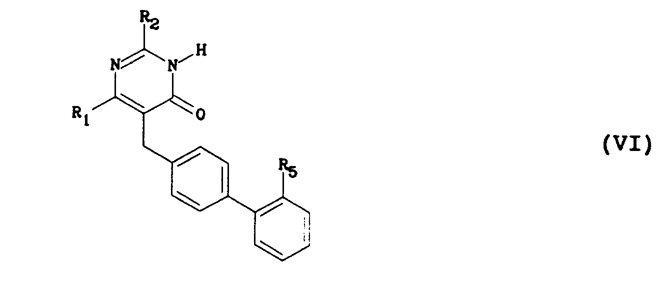 dans laquelle R₁, R₂ et R₅ sont tels que définis dans la revendication 1 et ci-dessus, puis on déprotège et/ou on transforme le groupe R₅, suivant la nature de ce groupe, pour obtenir, après transformation, un composé de formule générale (I).

4. Procédé selon la revendication 3, caractérisé en ce que l'on soumet les composés de formule générale (VI), dans laquelle R₅ est le groupe triphénylméthyl-1*H*-tétrazol-5-yle ou le groupe 1,1-diméthyléthyle-1*H*-tétrazol-5-yle et R₁ et R₂ sont tels que définis dans la revendication 1, à une réaction de déprotection en milieu acide, pour obtenir les composés de formule générale (I), dans laquelle R₃ est le groupe 1*H*-tétrazol-5-yle et R₁ et R₂ sont tels que définis dans la revendication 1.

5. Procédé selon la revendication 3, caractérisé en ce que l'on soumet les composés de formule générale (VI), dans laquelle R₅ est un groupe ester d'acide carboxylique, et R₁ et R₂ sont tels que définis dans la revendication 1, à une hydrolyse acide ou basique pour obtenir les composés de formule générale (I) dans laquelle R₁ et R₂ sont tels que définis dans la revendication 1 et R₃ est un groupe CO₂H.

6. Médicament, caractérisé en ce qu'il contient un composé selon l'une quelconque des revendications 1 et 2.

7. Composition pharmaceutique, caractérisée en ce qu'elle contient un composé selon l'une quelconque des revendications 1 et 2, en association avec tout excipient approprié.

## Revendications (Revendications pour l'(les) Etat(s) contractant(s) suivant(s): ES, GR)

1. Procédé de préparation de composés, existant sous trois formes tautomères, répondant aux formules générales (I), (I') et (I'') 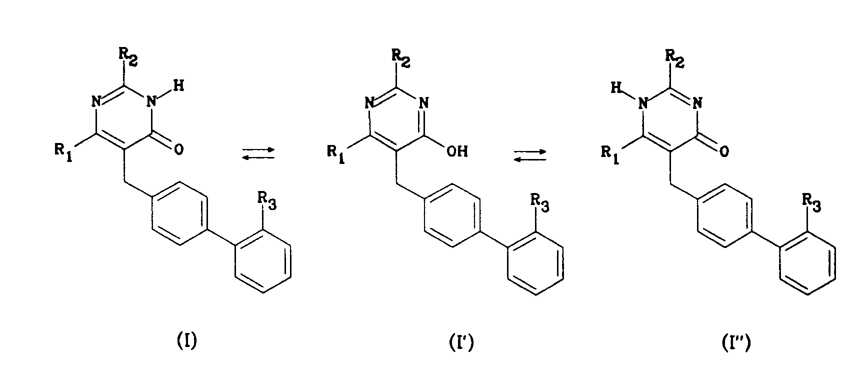 dans lesquelles
R₁ représente un groupe droit butyle,
R₂ représente un groupe phénylméthyle ou phényléthyle et
R₃ représente un groupe CO₂H ou 1*H*-tétrazol-5-yle,
ainsi que de leurs sels organiques ou inorganiques pharmaceutiquement acceptables,
procédé caractérisé en ce que l'on fait réagir un β-cétoester de formule générale (II) 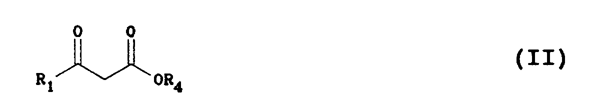 dans laquelle R₁ est tel que défini ci-dessus et R₄ représente un groupe méthyle ou éthyle, avec un dérivé de formule générale (III) 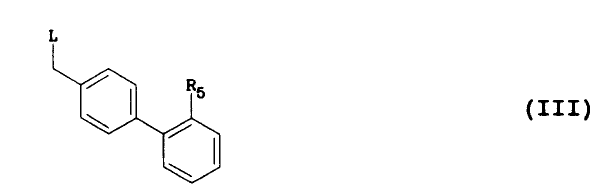 dans laquelle
L représente un groupe partant et R₅ représente soit un groupe CO₂R₆, où R₆ est un groupe méthyle, éthyle, 1,1-diméthyléthyle ou benzyle,
soit un groupe 1*H*-tétrazol-5-yle protégé,
pour obtenir un composé de formule générale (IV) 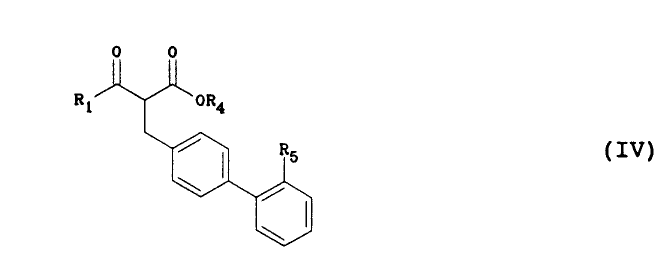 dans laquelle R₁, R₄ et R₅ sont tels que définis ci-dessus, composé que l'on fait réagir avec une amidine de formule générale (V) 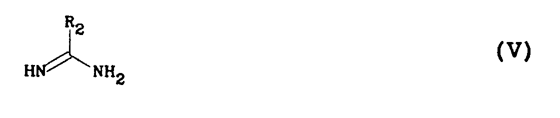 dans laquelle R₂ est tel que défini ci-dessus pour obtenir un composé de formule générale (VI) 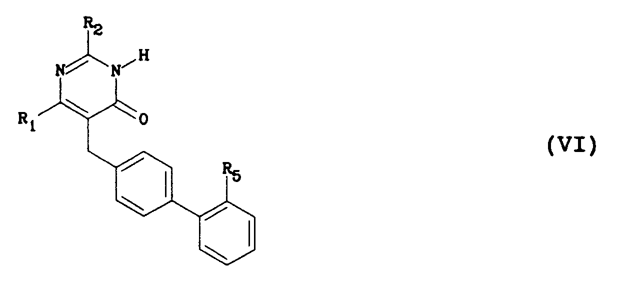 dans laquelle R₁, R₂ et R₅ sont tels que définis ci-dessus, puis on déprotège et/ou on transforme le groupe R₅, suivant la nature de ce groupe,
pour obtenir, après transformation, un composé de formule générale (I).

2. Procédé selon la revendication 1, caractérisé en ce que l'on soumet les composés de formule générale (VI), dans laquelle R₅ est le groupe triphénylméthyl-1*H*-tétrazol-5-yle ou le groupe 1,1-diméthyléthyle-1*H*-tétrazol-5-yle et R₁ et R₂ sont tels que définis dans la revendication 1, à une réaction de déprotection en milieu acide, pour obtenir les composés de formule générale (I), dans laquelle R₃ est le groupe 1*H*-tétrazol-5-yle et R₁ et R₂ sont tels que définis dans la revendication 1.

3. Procédé selon la revendication 1, caractérisé en ce que l'on soumet les composés de formule générale (VI), dans laquelle R₅ est un groupe ester d'acide carboxylique, et R₁ et R₂ sont tels que définis dans la revendication 1, à une hydrolyse acide ou basique pour obtenir les composés de formule générale (I) dans laquelle R₁ et R₂ sont tels que définis dans la revendication 1 et R₃ est un groupe CO₂H.

4. Procédé selon la revendication 1, caractérisé en ce que l'on prépare le 6-butyl-2-(2-phényléthyl)-5-[[2'-(1*H*-tétrazol-5-yl)[1,1'-biphényl]-4-yl]méthyl]-pyrimidin-4(1*H*)-one ainsi que ses sels organiques ou inorganiques pharmaceutiquement acceptables.

## Claims (Claims for the following Contracting State(s): AT, BE, CH, DK, DE, FR, GB, IT, LI, LU, MC, NL, PT, SE)

1. Compounds, existing in three tautomeric forms, corresponding to the general formulae (I), (I') and (I'') 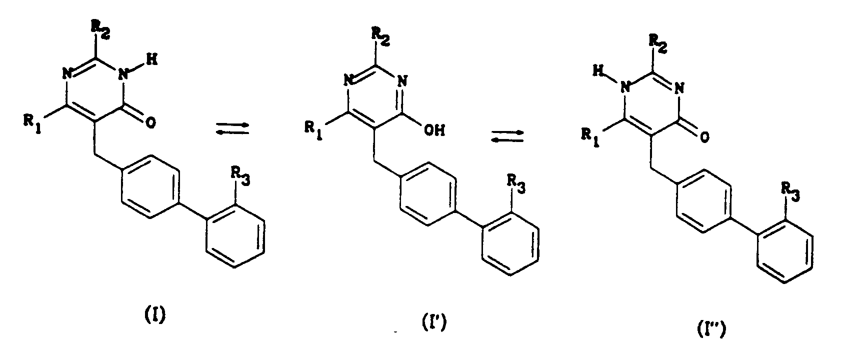 in which
R₁ represents a linear butyl group,
R₂ represents a phenylmethyl or phenylethyl group, and
R₃ represents a CO₂H or 1*H*-tetrazol-5-yl group,
as well as their pharmaceutically acceptable organic or inorganic salts.

2. 6-Butyl-2-(2-phenylethyl)-5-[[2'-(1*H*-tetrazol-5-yl)-[1,1'-biphenyl]-4-yl]methyl]-4(1*H*)-pyrimidinone, as well as its pharmaceutically acceptable organic or inorganic salts, according to Claim 1.

3. Process for preparing the compounds according to Claim 1, which process is characterised in that a β-keto ester of general formula (II) 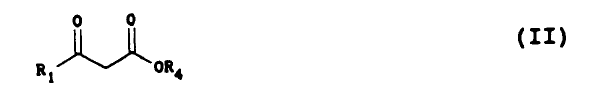 in which R₁ is as defined in Claim 1 and R₄ represents a methyl or ethyl group, is reacted with a derivative of general formula (III) 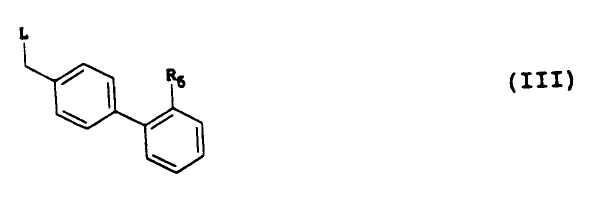 in which
L represents a leaving group and R₅ represents either a group CO₂R₆, in which R₆ is a methyl, ethyl, 1,1-dimethylethyl or benzyl group,
or a protected 1*H*-tetrazol-5-yl group,
to obtain a compound of general formula (IV) 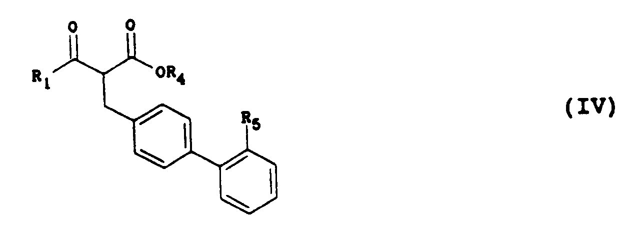 in which R₁, R₄ and R₅ are as defined in Claim 1 and above,
which compound is reacted with an amidine of general formula (V) 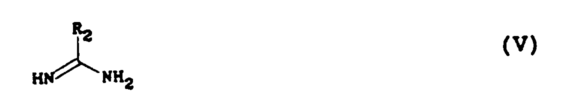 in which R₂ is as defined in Claim 1, to obtain a compound of general formula (VI) 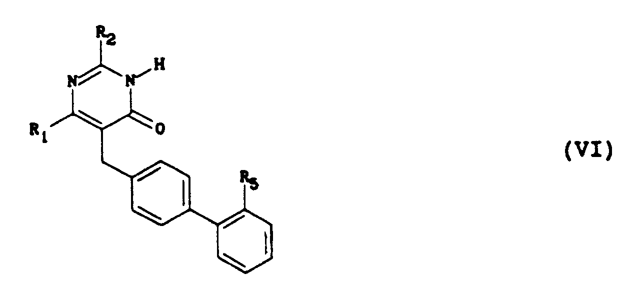 in which R₁, R₂ and R₅ are as defined in Claim 1 and above, and the group R₅ is then deprotected and/or converted in accordance with the nature of this group, to obtain, after conversion, a compound of general formula (I).

4. Process according to Claim 3, characterised in that the compounds of general formula (VI) in which R₅ is a triphenylmethyl-1*H*-tetrazol-5-yl group or a 1,1-dimethylethyl-1*H*-tetrazol-5-yl group and R₁ and R₂ are as defined in Claim 1 are subjected to a deprotection reaction in an acid medium, to obtain the compounds of general formula (I) in which R₃ is a 1*H*-tetrazol-5-yl group and R₁ and R₂ are as defined in Claim 1.

5. Process according to Claim 3, characterised in that the compounds of general formula (VI) in which R₅ is a carboxylic acid ester group and R₁ and R₂ are as defined in Claim 1 are subjected to an acid or alkaline hydrolysis, to obtain the compounds of general formula (I) in which R₁ and R₂ are as defined in Claim 1 and R₃ is a CO₂H group.

6. Medicinal product, characterised in that it contains a compound according to either of Claims 1 and 2.

7. Pharmaceutical composition, characterised in that it contains a compound according to either of Claims 1 and 2, in combination with any suitable excipient.

## Claims (Claims for the following Contracting State(s): ES, GR)

1. Process for preparing compounds, existing in three tautomeric forms, corresponding to the general formulae (I), (I') and (I'') 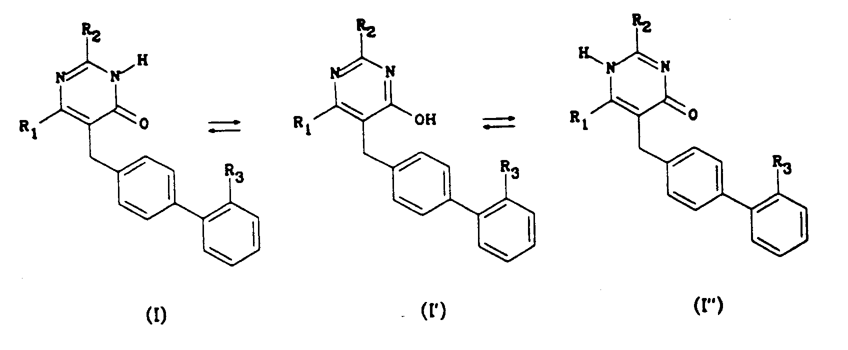 in which
R₁ represents a linear butyl group,
R₂ represents a phenylmethyl or phenylethyl group, and
R₃ represents a CO₂H or 1*H*-tetrazol-5-yl group,
as well as their pharmaceutically acceptable organic or inorganic salts, which process is characterised in that a β-keto ester of general formula (II) 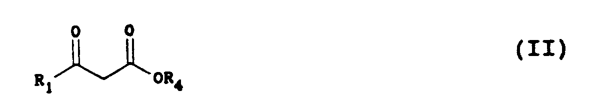 in which R₁ is as defined above and R₄ represents a methyl or ethyl group, is reacted with a derivative of general formula (III) 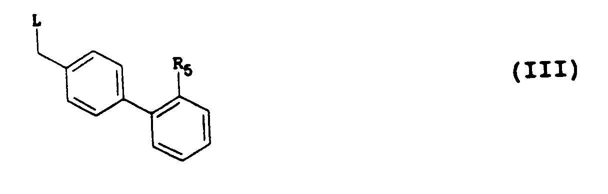 in which
L represents a leaving group and R₅ represents either a group CO₂R₆, in which R₆ is a methyl, ethyl, 1,1-dimethylethyl or benzyl group,
or a protected 1*H*-tetrazol-5-yl group,
to obtain a compound of general formula (IV) 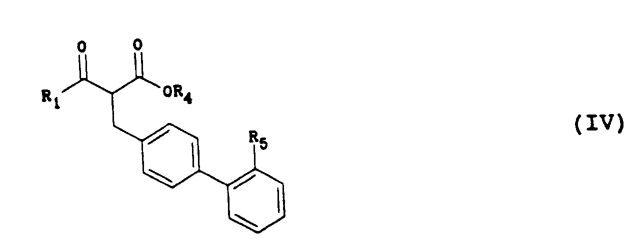 in which R₁, R₄ and R₅ are as defined above, which compound is reacted with an amidine of general formula (V) 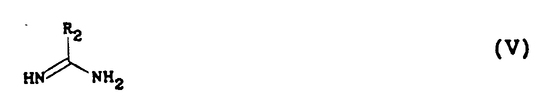 in which R₂ is as defined above, to obtain a compound of general formula (VI) 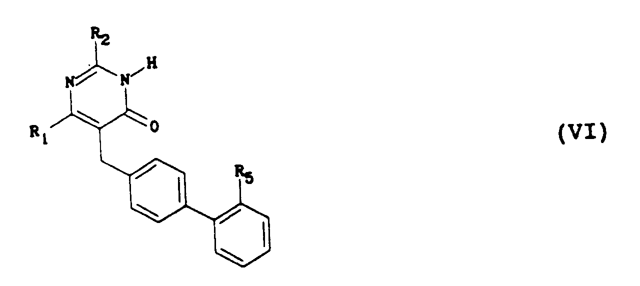 in which R₁, R₂ and R₅ are as defined above, and the group R₅ is then deprotected and/or converted, in accordance with the nature of this group,
to obtain, after conversion, a compound of general formula (I).

2. Process according to Claim 1, characterised in that the compounds of general formula (VI) in which R₅ is a triphenylmethyl-1*H*-tetrazol-5-yl group or a 1,1-dimethylethyl-1*H*-tetrazol-5-yl group and R₁ and R₂ are as defined in Claim 1 are subjected to a deprotection reaction in an acid medium, to obtain the compounds of general formula (I) in which R₃ is a 1*H*-tetrazol-5-yl group and R₁ and R₂ are as defined in Claim 1.

3. Process according to Claim 1, characterised in that the compounds of general formula (VI) in which R₅ is a carboxylic acid ester group and R₁ and R₂ are as defined in Claim 1 are subjected to an acid or alkaline hydrolysis, to obtain the compounds of general formula (I) in which R₁ and R₂ are as defined in Claim 1 and R₃ is a CO₂H group.

4. Process according to Claim 1, characterised in that 6-butyl-2-(2-phenylethyl)-5-[[2'-(1*H*-tetrazol-5-yl)-[1,1'-biphenyl]-4-yl]methyl]-4(1*H*)-pyrimidinone is prepared, as well as its pharmaceutically acceptable organic or inorganic salts.

## Patentansprüche (Patentansprüche für folgende(n) Vertragsstaat(en): AT, BE, CH, DK, DE, FR, GB, IT, LI, LU, MC, NL, PT, SE)

1. Verbindungen, die in drei tautomeren Formen der allgemeinen Formeln (I), (I') und (I'') vorkommen können: 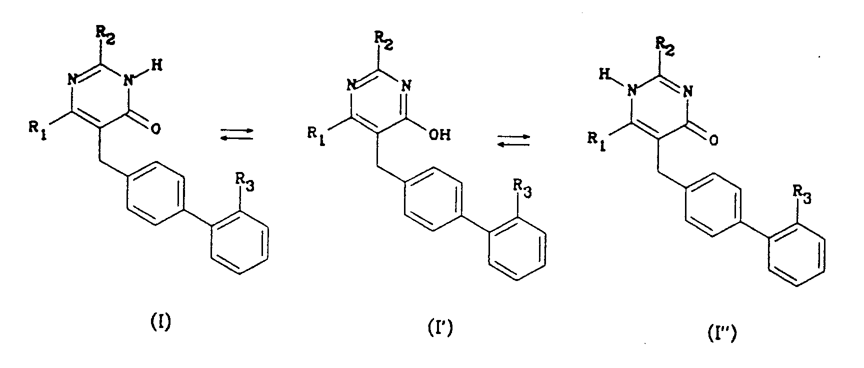 in denen
R₁ eine geradkettige Butylgruppe,
R₂ eine Phenylmethylgruppe oder eine Phenylethylgruppe und
R₃ eine CO₂H- oder 1H-Tetrazol-5-yl-gruppe bedeuten,
sowie deren pharmazeutisch annehmbare organische oder anorganische Salze.

2. 6-Butyl-2-(2-phenylethyl)-5-[[2'-(1H-tetrazol-5-yl)-[1,1'-biphenyl]-4-yl]-methyl]-pyrimidin-4(1H)-on sowie dessen pharmazeutisch annehmbare organische oder anorganische Salze nach Anspruch 1.

3. Verfahren zur Herstellung der Verbindungen nach Anspruch 1, **dadurch gekennzeichnet**, daß man einen β-Ketoester der allgemeinen Formel (II) 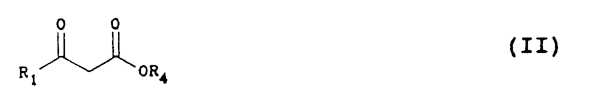 in der R₁ die in Anspruch 1 angegebenen Bedeutungen besitzt und R₄ eine Methyl- oder Ethylgruppe darstellt, mit einem Derivat der allgemeinen Formel (III) 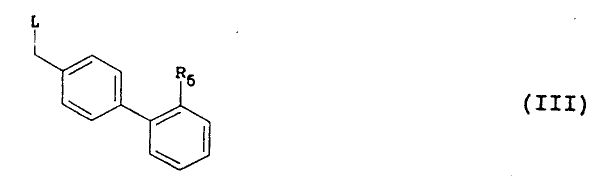 in der
L eine austretende Gruppe und R₅
entweder eine Gruppe CO₂R₆, worin R₆ eine Methyl-, Ethyl-, 1,1-Dimethylethyl- oder Benzylgruppe darstellt,
oder eine geschützte 1H-Tetrazol-5-yl-gruppe bedeuten,
umsetzt zur Bildung einer Verbindung der allgemeinen Formel (IV) 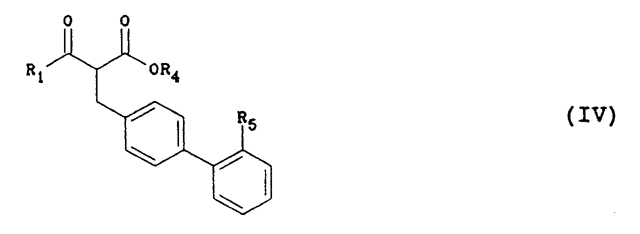 in der R₁, R₄ und R₅ die in Anspruch 1 und oben angegebenen Bedeutungen besitzen,
welche Verbindung man mit einem Amidin der allgemeinen Formel (V) 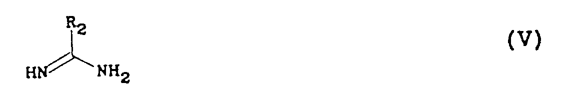 in der R₂ die in Anspruch 1 angegebenen Bedeutungen besitzt, umsetzt zur Bildung einer Verbindung der allgemeinen Formel (VI) 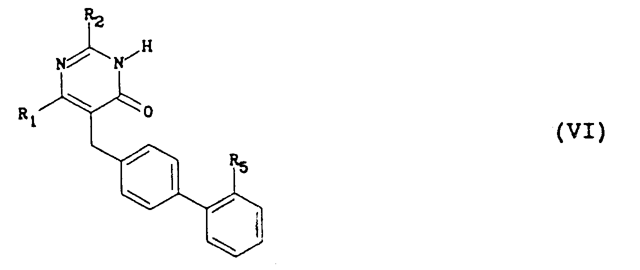 in der R₁, R₂ und R₅ die in Anspruch 1 und oben angegebenen Bedeutungen besitzen, und man die Schutzgruppe von der Gruppe R₅ abspaltet und/oder die Gruppe R₅ in Abhängigkeit von der Art dieser Gruppe umwandelt, so daß man nach der Umwandlung eine Verbindung der allgemeinen Formel (I) erhält.

4. Verfahren nach Anspruch 3, **dadurch gekennzeichnet**, daß man die Verbindungen der allgemeinen Formel (VI), in der R₅ die Triphenylmethyl-1H-tetrazol-5-yl-gruppe oder die 1,1-Dimethylethyl-1H-tetrazol-5-yl-gruppe darstellt und R₁ und R₂ die in Anspruch 1 angegebenen Bedeutungen besitzen, einer Reaktion zur Abspaltung der Schutzgruppe in saurem Medium unterwirft zur Bildung der Verbindungen der allgemeinen Formel (I), in der R₃ die 1H-Tetrazol-5-yl-gruppe darstellt und R₁ und R₂ die in Anspruch 1 angegebenen Bedeutungen besitzen.

5. Verfahren nach Anspruch 3, **dadurch gekennzeichnet**, daß man die Verbindungen der allgemeinen Formel (VI), in der R₅ eine Carbonsäureestergruppe darstellt und R₁ und R₂ die in Anspruch 1 angegebenen Bedeutungen besitzen. einer sauren oder basischen Hydrolyse unterwirft zur Bildung der Verbindungen der allgemeinen Formel (I), in der R₁ und R₂ die in Anspruch 1 angegebenen Bedeutungen besitzen und R₃ eine CO₂H-Gruppe darstellt.

6. Arzneimittel, **dadurch gekennzeichnet**, daß es eine Verbindung nach einem der Ansprüche 1 und 2 enthält.

7. Pharmazeutische Zubereitung, **dadurch gekennzeichnet**, daß sie eine Verbindung nach einem der Ansprüche 1 und 2 in Kombination mit irgendeinem geeigneten Trägermaterial enthält.

## Patentansprüche (Patentansprüche für folgende(n) Vertragsstaat(en): ES, GR)

1. Verfahren zur Herstellung von Verbindungen, die in drei tautomeren Formen der allgemeinen Formeln (I), (I') und (I'') vorliegen können: 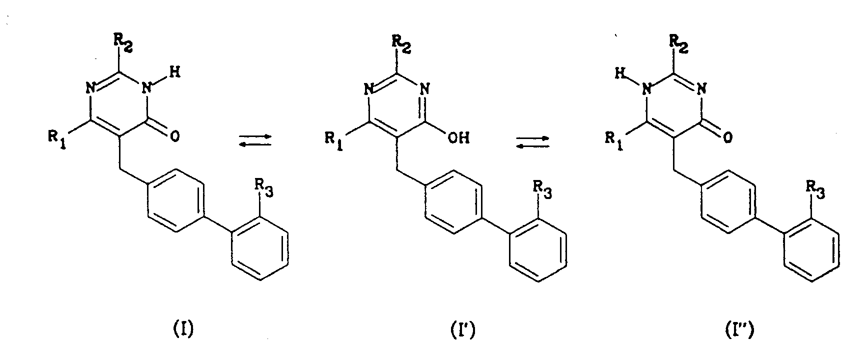 in denen
R₁ eine geradkettige Butylgruppe,
R₂ eine Phenylmethylgruppe oder eine Phenylethylgruppe und
R₃ eine CO₂H- oder 1H-Tetrazol-5-yl-gruppe bedeuten,
sowie von deren pharmazeutisch annehmbaren organischen oder anorganischen Salzen, **dadurch gekennzeichnet**, daß man einen β-Ketoester der allgemeinen Formel (II) 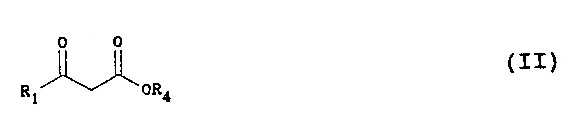 in der R₁ die oben angegebenen Bedeutungen besitzt und R₄ eine Methyl- oder Ethylgruppe darstellt, mit einem Derivat der allgemeinen Formel (III) 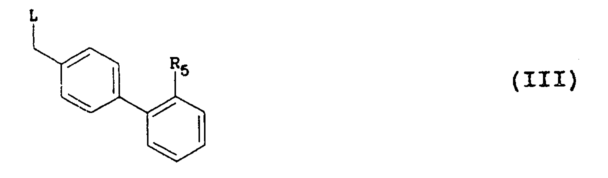 umsetzt, in der
L eine austretende Gruppe und R₅
entweder eine Gruppe CO₂R₆, worin R₆ eine Methyl-, Ethyl-, 1,1-Dimethylethyl- oder Benzylgruppe darstellt,
oder eine geschützte 1H-Tetrazol-5-yl-gruppe bedeuten,
zur Bildung einer Verbindung der allgemeinen Formel (IV) 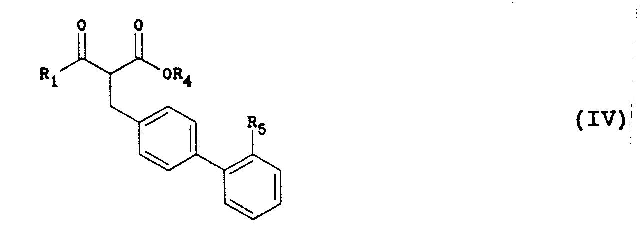 in der R₁, R₄ und R₅ die oben angegebenen Bedeutungen besitzen, welche Verbindung man mit einem Amidin der allgemeinen Formel (V) 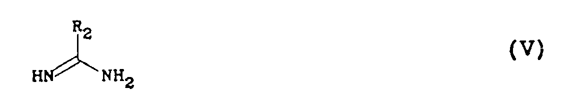 in der R₂ die oben angegebenen Bedeutungen besitzt, umsetzt zur Bildung einer Verbindung der allgemeinen Formel (VI) 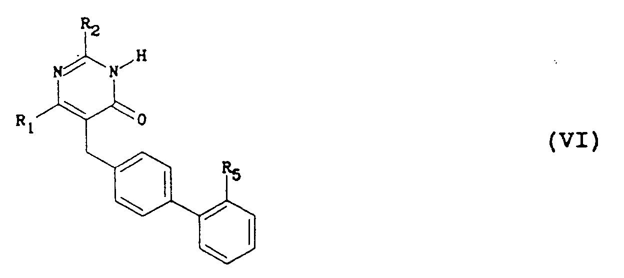 in der R₁, R₂ und R₅ die in oben angegebenen Bedeutungen besitzen, wonach man die Schutzgruppe von der Gruppe R₅ abspaltet und/oder die Gruppe R₅ in Abhängigkeit von der Art der Gruppe umwandelt, so daß man nach der Umwandlung eine Verbindung der allgemeinen Formel (I) erhält.

2. Verfahren nach Anspruch 1, **dadurch gekennzeichnet**, daß man die Verbindungen der allgemeinen Formel (VI), in der R₅ die Triphenylmethyl-1H-tetrazol-5-yl-gruppe oder die 1,1-Dimethylethyl-1H-tetrazol-5-yl-gruppe darstellt und R₁ und R₂ die in Anspruch 1 angegebenen Bedeutungen besitzen, einer Reaktion zur Abspaltung der Schutzgruppe in saurem Medium unterwirft zur Bildung der Verbindungen der allgemeinen Formel (I), in der R₃ die 1H-Tetrazol-5-yl-gruppe darstellt und R₁ und R₂ die in Anspruch 1 angegebenen Bedeutungen besitzen.

3. Verfahren nach Anspruch 1, **dadurch gekennzeichnet**, daß man die Verbindungen der allgemeinen Formel (VI), in der R₅ eine Carbonsäureestergruppe darstellt und R₁ und R₂ die in Anspruch 1 angegebenen Bedeutungen besitzen, einer sauren oder basischen Hydrolyse unterwirft zur Bildung der Verbindungen der allgemeinen Formel (I), in der R₁ und R₂ die in Anspruch 1 angegebenen Bedeutungen besitzen und R₃ eine CO₂H-Gruppe darstellt.

4. Verfahren nach Anspruch 1, **dadurch gekennzeichnet**, daß man 6-Butyl-2-(2-phenylethyl)-5-[[2'-(1H-tetrazol-5-yl)-[1,1'-biphenyl]-4-yl]-methyl]-pyrimidin-4(1H)-on sowie dessen pharmazeutisch annehmbare organische oder anorganische Salze herstellt.
